# EUROPEAN PATENT SPECIFICATION

(11) **EP 3 185 921 B1**
(45) Date of publication and mention of the grant of the patent: **04.09.2019**
(21) Application number: 15767421.9
(22) Date of filing: 25.08.2015
(51) Int. Cl.: A61L 29/14, A61L 33/00, A61M 1/02, A61M 5/14, B05D 3/14

(54) **METHOD FOR TREATMENT OF TOOLS AND TOOLS USED FOR ISOLATION OF MICROVESICLES, NANOVESICLES OR EXOSOMES**
VERFAHREN ZUR BEHANDLUNG VON WERKZEUGEN UND WERKZEUGE ZUR ISOLIERUNG VON MIKROVESIKELN, NANOVESIKELN ODER EXOSOMEN
PROCÉDÉ POUR LE TRAITEMENT D'OUTILS ET OUTILS UTILISÉS POUR L'ISOLEMENT DE MICROVÉSICULES, DE NANOVÉSICULES OU D'EXOSOMES

(30) Priority: 26.08.2014 GB 201415090
(43) Date of publication of application: 05.07.2017
(73) Proprietor: Jozef Stefan Institute, 1000 Ljubljana (SI); Univerza v Ljubljani, 1000 Ljubjana (SI)
(72) Inventor: JUNKAR, Ita, 1000 Ljubljana (SI); MOZETIC, Miran, 1000 Ljubljana (SI); ZAPLOTNIK, Rok, 1000 Ljubljana (SI); KRALJ-IGLIC, Veronika, 1000 Ljubljana (SI); STUKELJ, Roman, 1000 Ljubljana (SI)
(74) Representative: Marton, Dan-Robert
(86) International application number: PCT/EP2015/069420
(87) International publication number: WO 2016/030358

(56) References cited:
- US-A1- 2006 162 740
- US-A1- 2007 225 785
- US-A1- 2010 237 043
- A S CHIPER ET AL: "A COMPARATIVE STUDY OF HELIUM AND ARGON DBD PLASMAS SUITABLE FOR THERMOSENSITIVE MATERIALS PROCESSING", ROM. JOURN. PHYS, vol. 56, no. suppl, 1 January 2011 (2011-01-01), pages 126-131, XP055230267,

## Description

### FIELD OF THE INVENTION

The present invention relates to methods of preventing adhesion of micro vesicles onto tools used for collecting, sampling, storage, transport and isolation of microvesicles from mammal blood, as well as materials and products produced thereby. The mammal may be human, equine, bovine, murine, feline, porcine, canine or ovine. Preferably, the mammal is a human.

### BACKGROUND OF THE INVENTION

Atherosclerosis, coronary diseases, diabetes, inflammatory diseases, infectious diseases wound healing, neurologic diseases and metastasis of cancer represent a major health issue worldwide. While a variety of drugs have been invented the success rate in many cases is still not satisfactory. The main reason for rather unsatisfactory healing rates especially in case of cancer and inflammatory diseases are due to the fact that drugs are often used when diseases have already matured. Much more successful healing rates are expected if drugs are taken in the early stage of the disease. Many of these diseases are, however, difficult to diagnose in early stage using current techniques, so novel alternative techniques for early diagnosis should be invented. Many studies have shown that microvesicles are involved in cell to cell communication by transfer of bioactive molecules including proteases, mRNAs, microRNAs, membrane receptors and thus play an important role in physiological/pathological pathways including progression of various diseases (Simak et al. 2006, Cocucci et al.2009, Record et al. 2011) and are increasingly cited as potential biomarkers. Recently it has been confirmed that the fingerprint of initial cancer stages is found in blood as well as cerebrospinal fluids in the form of small subcellular structures called microvesicles or nanovesicles. Smallest versions of subcellular structures are often called exosomes. All membrane vesicles shed by cells are herein collectively referred as microvesicles.

Typically the microvesicles' size range is between 100 and 1500 nm, while the size of exosomes is in the range of 30 to 100 nm. They are small sub-cellular structures, which are surrounded by a phospholipid bilayer and are released into the extracellular environment from most cell types, including tumour cells. Microvesicles are mainly formed by blabbing of the plasma membrane, while exosomes are released by exocytosis from multivesicular bodies of endosome (Cocucci et al. 2009, Muralidharan-Chari et al. 2010). Because microvesicles carry with them nucleic acids from their cell-of-origin the diagnostic of micro vesicles can provide real-time access to molecular genetic information of cells in the body without the need to have a direct access to the actual cells. This is especially important since the micro vesicles carry information on the existence of cancer cells well before they develop into tumours and it is especially beneficial in cases where direct cellular biopsy may be difficult or otherwise unattainable. Moreover, in the biopsy procedures, tissue samples are taken from a limited area and may give false results, especially in tumours which are heterogeneous and/or dispersed within normal tissue. Isolation of microvesicles can be used also as molecularly-targeted therapy, which is also gaining its importance in cancer therapy. Therefore characterization and isolation of microvesicles is a promising technique which could give information regarding early stages of different diseases as well as provide development of different therapeutic treatments. Thus microvesicles derived from B-cells and dendritic cells are stated to have potent immune-stimulatory and antitumour effects in vivo and have been used as antitumor vaccines (Chaput et al. 2005). The vesicles, however, are not abound in human and should be therefore isolated carefully to gain satisfactory concentration for diagnostics or therapeutic purposes. Isolation of microvesicles currently represents a major drawback due to a simple fact that they are likely to interact with surfaces of medical tools, especially surfaces of Eppendorf tubes where blood or cerebrospinal fluid is stored. The interaction leads to depletion of the microvesicles in liquids so their concentration usually falls below the detection limit merely by irreversible sticking onto the surface of the Eppendorf tubes or other medical tools used for collecting, sampling, storage, transport and isolation of microvesicles. Isolation and characterization of microvesicles includes ultracentrifugation, differential centrifugation (Raposo et al. 1996), ultrafiltration (Cheruvanky et al., 2007), size chromatography, anion exchange and/or gel permeation chromatography (US 6899863), general protein assay as well as affinity purification with antibodies (Vlassov et al. 2012, US 20090220944). Methods of sucrose density gradients or organelle electrophoresis are described in US 7198923. The isolation by organelle electrophoresis comprises the separation of exosomes from blood plasma of an individual infected with hepatitis C virus and the extraction of RNA from these exosomes.

Microvesicles can also be purified and isolated by microchip technology based on the unique microfluidic platform which efficiently and selectively separates tumour derived microvesicles (Nagrath et al., 2007). To isolate and/or enrich of microvesicles isolated from body fluids from specific cell types (lung, stomach, breast, liver etc.) surface molecules such as antigens from their donor cells can be used (Al-Nedawi et al., 2008). It has been shown that tumour (malignant and non-malignant) microvesicles carry tumour associated surface antigens.

Expression analysis using microarrays and ultra-deep sequencing in cancer and other indications reveals a large number of both coding and non-coding RNA biomarkers available for analysis. It has been stated that gene expression profiles can distinguish between cancerous and non-cancerous tissue (Jones et al., 2008, Parsons et al., 2008). However it has become now apparent that traditionally used techniques to both isolate and characterise biological materials are not suitable or have not been developed and refined to work with microvesicles. Depending on the biofluid medium and the nucleic acid target of interest, successive levels of enrichment can be achieved by applying various isolation technologies to increase sensitivity. Current methods used to increase concentrations of microvesicles are achieved by large impute volumes and lengthy enrichment processes. However in many cases increased sensitivity is impossible to obtain with standard isolation techniques, which hampers the development and discovery of this potentially broad area of research. Therefore in future significant efforts will be made to design novel isolation techniques, which will allow for higher detection and by this better diagnostics of microvesicles.

Methods for isolation and detection of microvesicles are well known in the arts. Usually these methods include series of differential centrifugation steps to separate cells or cell debris from the biological media (Raposo et al. 1996).

A method for preparing microvesicles from biological sample by anion-exchange chromatography is described in WO0044389. The method enables preparation of microvesicles and its separation from other biological contaminants by at least one anion-exchange chromatography and gel permeation chromatography. By this method the enrichment process of microvesicles is done by a step of clarification and a subsequent stage of concentration which is achieved by affinity chromatography, centrifugation at low rate and/or filtration.

Methods of producing, purifying, detecting or using exosomes for therapeutic purposes or as research tool have been described in WO2013084000, WO 2012087241 or WO 2012108842.

Novel methods for producing microvesicles in high yields, high purity and relatively short preparation times by density cushion centrifugation and a series of ultrafiltration steps and/or clarification step is disclosed in US 2004241176.

In the field of cancer diagnosis the method to quantify and qualify microvesicles is described (US 2009220944). This disclosure shows that ELISA based test (called Exo Test) enables quantification and characterization of microvesicles (exosomes) from human plasma of both healthy donors and tumour patients.

Exosomes may be also purified using antibodies specific to particular exosome epitopes as disclosed in US2009220944.

In US2013323756 methods and compositions for quantifying exosomes are disclosed. Embodiments of this invention are based on methods, composition and kits that utilize lectins to quantify exosomes.

Methods of treatment for medical tools to improve their surface properties are also known in the art.

The method of treatment of certain cylindrical objects made of polymers which can be used in medicine, biology, chemistry, pharmacy, material science and industry is disclosed in patent RO 128576. The invention is based on the radiofrequency plasma generating device which comprises a tube of dielectric material open at both ends where-through the treated object gets in, outside of discharge there are two ring-shaped metal electrodes (power electrode, mass electrode). The device may also use a series of alternately polarized electrodes, while the working gas is introduced into the tube through an insertion placed between the two electrodes and it comes out through the tubes ends, the discharge is taking place only inside the tube.

In EP 0348969 a method for rapid adherence of endothelial cells onto a surface and surfaces prepared thereby is disclosed. Small diameter plastic tubing is plasma-treated on the lumen wall for the purpose to increase endothelial cell attachment to give antithrom-bogenic small diameter conduits useful for vascular grafts and other articles intended to come in contact with blood.

Modification of surface material by: a. exposing the surface to a glow discharge plasma to activate the surface; b. exposing the surface to an ethylenically unsaturated monomer or mixture of monomers; and c. irradiating the activated surface with gamma radiation or electron beam radiation in the presence of the ethylenically unsaturated monomer to form a graft polymerized coating thereon is disclosed in US 5376400. The method by this invention acts to reduce cell and tissue abrasion and adhesion and to thereby reduce fibrous capsule formation. Such treatment can reduce thrombogenicity and improve biocompatibility of blood tubing, blood bags, catheters and other medical devices made of polyvinylchloride.

The invention which prevents the occurrence of blood coagulation and plasma protein deposition by applying corona discharge or glow discharge treatment to the surface of a molded product such as a film and a tube mainly composed of polypropylene and in contact with blood, and negatively charging the surface is disclosed in JPH0889570.

In US 2010/237043 A1 a method for plasma deposition of material, derived from plasma etching of a polymeric dielectric material, onto the surface of a substrate in order to increase adhesion of the substrate's surface is disclosed. The described process uses continuously produced plasma for etching and producing the deposition material in form of volatile byproducts.

Another method for increasing the adhesiveness of the surface of medical tools is described in US 2007/225785 A1. Therein medical devices having textured surfaces and methods for providing such devices are disclosed, whereby all texturing methods are based on a continuous exposure of said medical device to the plasma cloud within a plasma chamber.

Finally, in US 2006/162740 A1 a method and apparatus for cleaning and surface conditioning objects using non-equilibrium atmospheric plasma is disclosed. Since the described method aims explicitly at cleaning and sterilizing surfaces by using plasma, the obtained surface textures are supposed to be smooth in order to avoid re-contamination after the disclosed cleaning process.

Despite the various methods known for isolation and detection of microvesicles from body fluids the need to improve the currently used techniques still remains.

Therefore, one object of the present invention is to provide an improved method of treatment polymeric materials used for collecting, sampling, storing, transporting and isolating of microvesicles from fluids, in particular body fluids.

### SUMMARY OF THE INVENTION

The present invention is defined by the appended claims. The present invention relates to a method which prevents excessive adsorption of, for instance microvesicles but not limited thereto, on the surfaces of tools used for collecting, sampling, storing, transporting and isolating of body fluids containing microvesicles according to claim 1. The present invention also relates to materials, devices or tools produced (i.e., modified or treated) by the method according to the present invention, and to products produced from such materials.

According to the present invention a method which prevents excessive adsorption of microvesicles on the surfaces of tools used for sampling, storing and handling body fluids containing microvesicles is provided. The method comprises the steps of: selecting said a tool from the a list plurality of tools including but not limited to needles, blood tubing, blood bags, catheters, Eppendorf tubes, or pipettes, providing said tool from said plurality of tools, wherein said tool is made from a polymeric material, providing a source of highly ionized gas of positively and negatively charged particles of high density, selecting a source assuring for formation of positively and negatively charged particles of high density and treating a surface of said tool by applying short pulses of said source of particles next to or on the said surface of said tool to assure surface modification of said surface by reacting said positively and negatively charged particles of high density on said surface.

The method according to the present invention ensures contacting of tools with short pulses of highly ionized gas comprising both positively and negatively charged particles, the pulses being essentially short enough to avoid excessive heating of materials used for collecting, sampling, storage, transport and isolation of micro vesicles and the density of both positively and negatively charged particles which is essentially high enough to cause roughening of said tools on sub-micrometer or nanometer scale.

Especially tools treated according to the present inventive method prevents excessive adsorption of microvesicles on the surfaces of said tools used for collecting, sampling, storing, transporting and isolating of microvesicles or the like.

The method according to the present invention enables higher yields and lower fragmentation of microvesicles for instance by preventing adsorption of this valuable diagnostic material on the surface of different tools used for isolation and detection.

Accordingly, the present invention also provides increasing the roughness of a material by the method according to the present invention. In particular the material which is used to produce diagnostic or medical tools or devices.

Said tools used for sampling, storage and handling body fluids containing microvesicles are made from polymer materials.

In a preferred embodiment, said polymeric material is selected from the group consisting of biopolymers, synthetic polymers, polyethylene, polypropylene, polyurethane, polyterpene, inorganic polymers, phenolic resins, polyanhydrides, polypropylene, polystyrene, polyolefins, polyalkenes, polyamide, polyacetal, polyethylene terephthalate, polycarbonate, and cellulose acetate.

A particularly preferred polymeric material is polypropylene, polyethylene terephthalate and polystyrene.

In preferred embodiments of the invention, the tools used for collecting, sampling, storage, transport and isolation of microvesicles are selected from the group consisting but not limited to collecting tubes, tubes in use for centrifugation (Eppendorf tubes), pipette tips, long and narrow tubes in use as connectors for diagnostic purposes, culture plates, blood bags and implantable medical devices.

According to the invention, the pulse of both positively and negatively charged gaseous particles is of a length between 1 and 1000 ns, a ns (nano-second) being 1/1000000000 s (second).

In further preferred embodiments, the pulse of both positively and negatively charged gaseous particles is of a length between 10 and 100 ns, a ns (nano-second) being 1/1000000000 s (second).

According to the invention, the density of both positively and negatively charged gaseous particles in the pulse is essentially between about 10¹⁶ m⁻³ (10¹⁶ particles per cubic meter) and 10²⁰ m⁻³.

In further preferred embodiments, the density of both positively and negatively charged gaseous particles in the pulse is between about 1x10¹⁷ m⁻³ and 1x10¹⁹ m⁻³.

In further preferred embodiments, the density of both positively and negatively charged gaseous particles in the pulse is between about 3x10¹⁷ m⁻³ and 3x10¹⁸ m⁻³.

In preferred embodiments of the invention, the selected area of surface of tools used for collecting, sampling, storage, transport and isolation of microvesicles is subjected to a multiple pulses of both positively and negatively charged gaseous particles. The frequency of pulses is essentially below 1 MHz. This range of pulse frequency was observed as being very favourable leading to an increase of the yield while using the tools treated by the present method.

In further preferred embodiments of the invention, the selected area of surfaces of tools used for sampling, storage and handling body fluids containing micro vesicles is subjected to a number of pulses ranging from 100 to 10,000 pulses of both positively and negatively charged gaseous particles. The frequency of pulses is preferably between 0.1 and 100 kHz. Preferably, the contacting of said tools used for sampling, storage and handling body fluids containing microvesicles with said pulses of both positively and negatively charged gaseous particles is at a maximum bulk material temperature of between 20°C and 100°C, preferably between 20°C and 50°C, most preferred between 20° and 30°C. This is to ensure that no uncontrolled destruction of materials said tools used for collecting, sampling, storage, transport and isolation of microvesicles from body fluids are made, which may negatively affect the material's properties.

By the present methods of invention, the contacting or treating step is in a dry state, under atmospheric pressure.

Also described are polymeric materials modified by a method according to the present invention. Such material has advantageous properties, in particular, prevents adsorption of microvesicles. Preferably, the modified material of the invention is polypropylene, polyethylene terephthalate and polystyrene.

The present invention also relates to tools comprising the polymeric material of the invention. In preferred embodiments, the tool is selected from the group of products consisting but not limited to collecting tubes, tubes in use for centrifugation (Eppendorf tubes), pipette tips, long and narrow tubes in use as connectors for diagnostic purposes, culture plates, blood bags or implantable medical devices.

### BRIEF DESCRIPTION OF THE DRAWINGS

The invention is herein described, by way of example only, with reference to the accompanying drawings, wherein:
- Fig.1: shows a schematic of the surface effects according to a method of the invention;
- Fig. 2: visualizes the surface of an untreated sample (a) and a sample of polymeric material treated by method according to the invention (b);
- Fig. 3: visualizes the concentration of microvesicles in the body fluid stored in Eppendorf tubes treated by methods of invention and untreated Eppendorf tubes, wherein results from 2 patients one with celiac disease (sample 1) and one with diabetes (sample2) are shown;
- Fig. 4: visualizes the concentration of microvesicles and their fragments in Eppendorf tubes treated by the methods of invention (upper images) and untreated Eppendorf tubes (lower images); and
- Fig. 5: visualizes the concentration of microvesicles fragments below the 460 nm size obtained from flow cytometer, wherein results from 2 patients; one with celiac disease (sample 1) and one with diabetes (sample 2) after storage in Eppendorf tubes treated by methods of invention and untreated Eppendorf tubes are shown.

### DETAILED DESCRIPTION OF THE INVENTION

The expression "adhesion of microvesicles ", according to the present invention, shall be understood to define the tendency of a microvesicle to stick onto the surface of a material upon contacting with body fluid and remain bonded on the surface. Accordingly, the adhesion of microvesicles occurs on the surface of tools used for collecting, sampling, storage, transport and isolation of microvesicles from blood or cerebrospinal fluid. The adhesion occurs on the surface of any material contacted with blody fluid, preferably blood or cerebrospinal fluid containing microvesicles, in particular on surfaces of tools made from polymers, preferably but not limited to polypropylene, polyethylene terephthalate and polystyrene. The adhesion of microvesicles on surfaces causes depletion of microvesicles from the body fluid, what in turn leads to drop of concentration of microvesicles below the detection limit as well as higher fragmentation of microvesicles during isolation procedures.

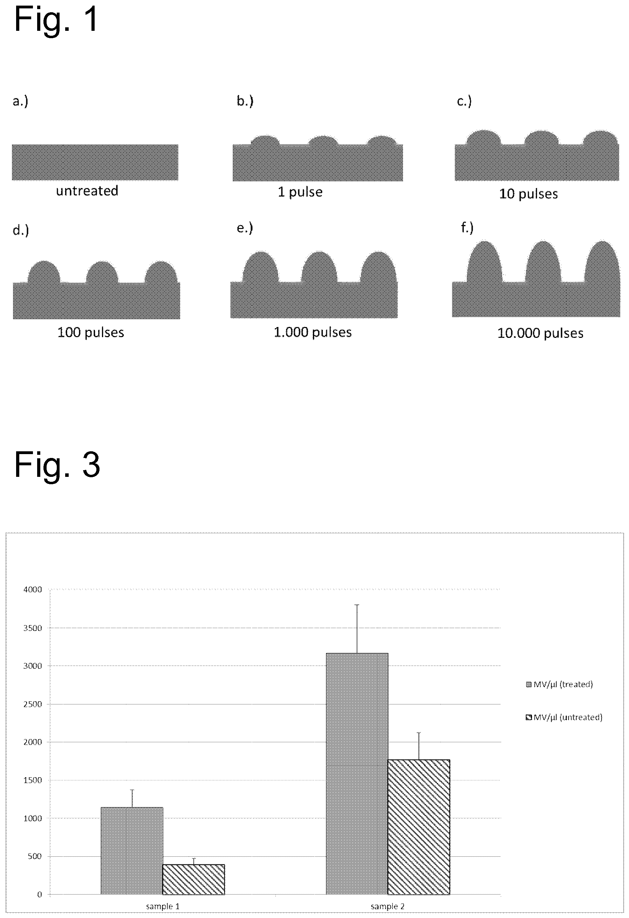

Fig. 1 represents schematically the effect of short pulses of highly ionized gas comprising both positively and negatively charged particles on the surface morphology of selected polymer on a sub-micrometer scale. The surface of tools used for collecting, sampling, storage, transport and isolation of microvesicles made from polymer materials are originally very smooth with no distinguished morphology. A confirmation of this statement is presented in Fig. 1 (a). This figure represents and AFM image of a fraction of an Eppendorf tube made from polypropylene. The surface area sampled by AFM is 1 µm x 1µm. The abbreviation AFM stands for Atomic Force Microscopy - a technique nowadays used widely in order to determine the surface morphology of solid materials on micrometer (µm) as well as sub-micrometer scale. Short pulses of highly ionized gas comprising both positively and negatively charged particles of duration 30 ns were applied in order to modify morphology of the polymer material. The charged particles neutralize upon contacting the polymer surface causing displacement of surface atoms. The surface atoms reorganize on the polymer surface but do not assume the original positions. Instead they form small bulges as presented schematically in Fig. 1. The formation of these bulges is governed by the surface energy of solid materials. Namely, once surface atoms and molecules are allowed to migrate on the surface they will form spherical shapes in order to satisfy the lowest free energy conditions. The time scale of the treatment with a single pulse should be essentially very short, short enough to prevent heating of the bulk polymer material. The typical time scale is solely 30 ns. Due to the large density of charged particles (preferably 1x10¹⁸ m⁻³) a huge power is dissipated. Taking into account typical ionization energy of a gaseous molecule or atom (10 eV) the average power over the pulse duration of 30 ns is about 50x10⁶ W (50 Mega Watt). Such a huge power would cause destruction of all materials if applied over a long time scale.

According to the methods of invention, however, such destruction of an Eppendorf tube does not occur due to the very short time scale of preferably 30 ns. Instead of destruction of the entire Eppendorf tube the power released at neutralization of the charged particles is transferred to the surface atoms and molecules. The surface layer of polymer material melts and partially evaporates upon treatment with the pulse of charged particles. The low-molecular fragments which evaporate from the surface of polymer material upon treatment with the pulse of charged particles interact with said charged particles leading to their dissociation and partial ionization. The molecules evaporated from the surface thus fragmentise to the constituents, i.e. C, H and CHₓ radicals who interact readily with ambient oxygen and finally form stable products like CO₂ and H₂O. The process described above is similar to laser ablation except that dissociation and ionization of evaporated molecules in the case of laser ablation is by far less efficient due to the fact that the cross-section for such reactions with laser photons is orders of magnitude smaller than with charged particles. If laser pulses are used for ablation the ablated material does not oxidize completely but is rather re-deposited in the form of soot so the final result of laser treatment is completely different than the treatment according to the methods of invention.

Treatment of polymer material with pulses of charged particles does not only lead to evaporation but also to melting a very thin surface layer of polymer. The high temperature in the molten film causes partial de-hydrogenation of the polymer so that the composition as well as structure of the surface film changes. De-hydrogenized material has essentially lower vapour pressure at given temperature than untreated material so it is slightly more resistant to evaporation that the untreated material. Oxygen in the ambient gas interacts with surface of molten material causing formation of oxygen-rich surface functional groups which in turn cause increased surface energy. Liquids of high surface energy spontaneously cause formation of droplets since it is energetically more favour than flat surface. If thick layer were melted the surface would probably become covered with droplets of partially de-hydrogenated material even after one single pulse of charged particles. Since the energy is dissipated only on the surface, however, only a very thin layer is melted and the majority of available power is spent for evaporation. That is why small bulges appear on the surface instead of droplets after a pulse of treatment with charged particles.

The partially de-hydrogenated material thus shrinks to small bulges upon treatment with the methods of invention as shown in Fig. 1 (b). The surface not covered with bulges retains its original composition and structure so it is more sensitive to further treatment with pulses of charged particles. More partially de-hydrogenated material is produced upon treatment so the bulges slowly increase in vertical dimension with increasing number of pulses. The lateral dimension of the bulges, however, remains fairly intact since the partially de-hydrogenated material shrinks spontaneously due to the large surface energy as explained above.

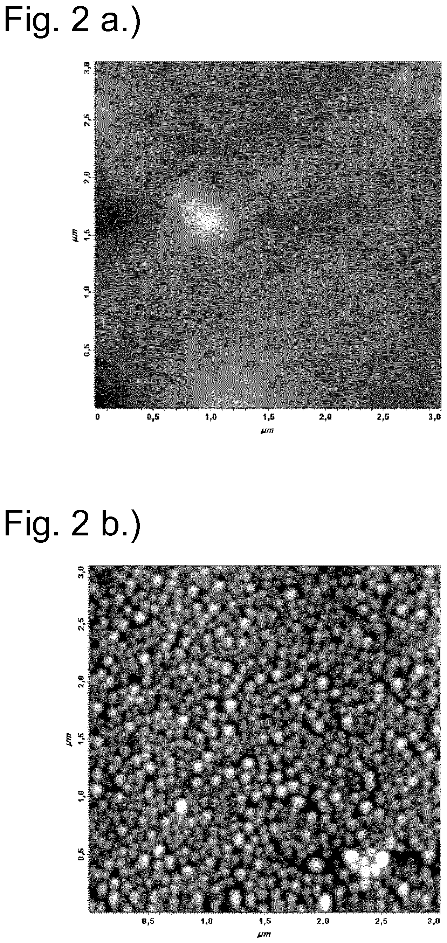

The bulges therefore grow with increasing number of pulses and eventually form cone-like structures are presented schematically in Fig. 1 (b) - (f). Finally the entire surface of the polymer becomes covered with dense cones what is schematically presented in Fig. 1 (f) and measured by AFM - Fig. 2 (b). Such self - organization of the partially de-hydrogenated material therefore allows for a drastic change of the surface morphology upon treatment of polymer material according to the methods of invention as revealed by comparing Fig. 2 (a) and 2 (b).

Surfaces of polymers with such morphology as presented in Fig. 2 (b) will not interact with body liquids such as human blood in the same manner as untreated materials. Flat surfaces represent infinite binding site for various blood particles including microvesicles, nanovesicles and/or exosomes (thereafter: microvesicles). Therefore, microvesicles adhere well onto the surface of untreated materials causing depletion of blood. In contrary, the surfaces of materials treated according to the methods of invention, represent limited binding sites for adhesion of microvesicles. Therefore, materials treated according to the methods of invention exhibit lower adhesion of microvesicles compared to untreated materials. The effect is supported with results of flow-citometry measurements of microvesicles stored in untreated Eppendorf tubes and Eppendorf tubes treated according to the methods of invention.

Fig. 3 shows quantitative results on the concentration of microvesicles and their fragments in untreated Eppendorf tubes and Eppendorf tubes treated by methods of the invention. Blood was taken from two patients; one with celiac disease (sample 1) and one with diabetes (sample 2). The concentration of microvesicles in blood stored in Eppendorf tubes treated according to the methods of invention is about two times larger than in blood stored in untreated Eppendorf tubes.

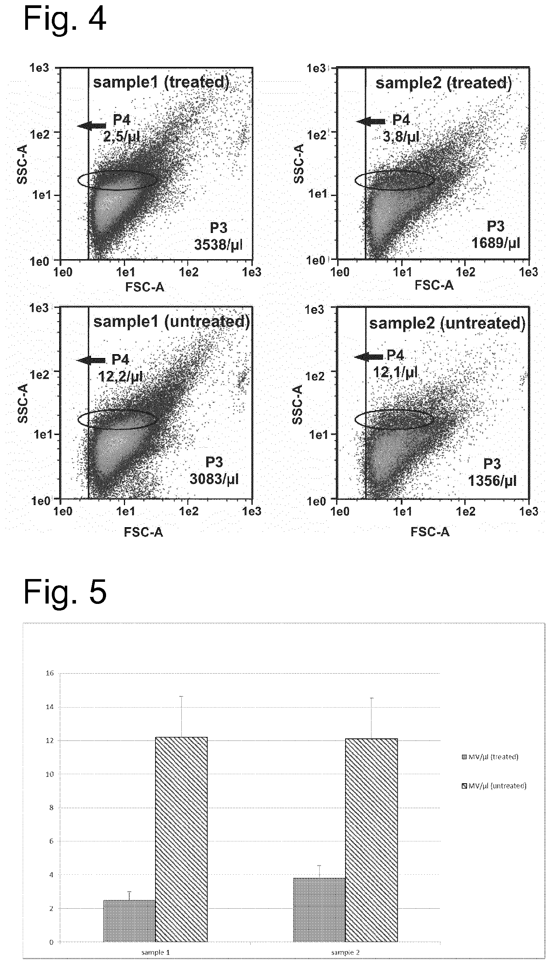

Fig. 4 represents the forward and side scattered parameters obtained with flow cytometer for microvesicles isolated from patients with celiac disease (sample 1) and diabetes (sample 2) after storage in untreated Eppendorf tube and treated Eppendorf tube according to the method of invention. The density plot according to Fig. 4 (forward scattered light FSC-A vs. side scattered light SSC-A) representing microvesicles of two volunteers with diferent diseases (sample 1-celiac disease; sample 2- diabetes). The region (P1) estimates events of small microvesicles, black region is representing a 460nm calibration beads, and a green region (P3) is a total number of microvesicles in the sample.

The region (P4) estimates events of small microvesicles, region (P3) is a total number of microvesicles in the sample. Elipse shape is representing a 460nm calibration beads for size comparisation.

The difference is in this case even more pronounced then in the case of total concentration of microvesicles. The methods of invention therefore not only reduce the loss of microvesicles due to surface adhesion but also minimize fragmentation of these particles mostly due to lower sticking on the Eppendorf tubes and thus lower induced mechanical stress on the microvesicles. The details about blood sampling and quantification of results will be presented in the following examples.

### Example 1 and 2.

Blood sampling was performed according to the Helsinki declaration considering research on humans, the Oviedo convention on human rights and biomedicine, and the Slovenian medical deontology codex. The study was approved by the Slovenian National Medical Ethics Committee, No 117/02/10. No adverse effects on donors health due to sampling were observed.

Two patients, one (male, 55 years) with celiac disease and one (male, 60 years) with diabetes diagnosis donated 5,4 ml of blood. Blood was withdrawn from the medial cubital vein with 21 gauge needle (MULTI 104 Sample Needle, Nipro Corporation, Tokyo, Japan) using two 2.7 mL vacutubes containing 270 µL of sodium citrate at a concentration 0.109 mol/L (Becton Dickinson, New Jersey, USA). Blood was immediately homogenized and evenly distributed into two 4ml tubes. Blood was centrifuged at 1550 g and 37°C for 20 ̌̌minutes in a Centric 200/R centrifuge (Domel d.o.o., Železniki, Slovenia). Upper 250 µL of plasma was slowly removed (using treated/untreated tips) and placed in a 1.8 mL treated and untreated Eppendorf microtube. The samples were then centrifuged at 17570 g for 5 minutes in a Centric 200/R centrifuge (Domel d.o.o., Železniki, Slovenia), so that microvesicles were gathered on the bottom of the microtube. The upper 210 µL of plasma was discarded and the remaining 40 µL of pellet was vortexed at 1200 g and resuspended in 210 µL of citrated phosphate-buffered saline (pH = 7.4). The samples were centrifuged again at 17570 g for 5 minutes. 210 µL of the supernatant was discarded and 40 µL pellet resuspended in 60 µL of citrated phosphate buffered saline (pH = 7.4). The concentration of microvesicles in isolates was determined by further analysed with MACSQuant Analyzer (Miltenyi Biotec GmbH, Bergisch-Gladbach, Germany) flow cytometer with 405 nm, 488 nm and 640 nm air cooled lasers. The MACSQuantifyTM 143 (Miltenyi Biotec GmbH, Bergisch-Gladbach, Germany) software version 2.4. was used for data acquisition and analysis of the results. 25 µL of sample was measured. With flow cytometers the presence of microvesicles was determined by forward and side scatter parameters. The results of these studies are presented in Fig. 3, where the concentration of microvesicles after storage in treated Eppendorf tubes by the method of invention and the untreated Eppendorf tube are presented for two patients (one with celiac disease and the other with diabetes). In Fig. 4 the density plot (forward scattered light FSC-A vs. side scattered light SSC-A) representing microvesicles of two volunteers with different diseases (sample 1-celiac disease; sample 2- diabetes) is presented. From this plot it is possible to observe significant difference in microvesicle size and concentration according to the different isolation technique (one stored in treated Eppendorf tubes by the method of invention and the other stored in untreated Eppendorf tubes). More pronounced differences can be observed in the distribution of microvesicles below the size of 460 nm (left side of the black line in Fig. 4). The samples stored in untreated Eppendorf tubes have much higher number of microvesicles in this side of the plot in comparison to the samples stored in Eppendorf tubes according to the method of invention. These is also presented in Fig. 5, where comparison of the concentration of microvesicles below the 460 nm size is shown for two patients (sample 1- with celiac disease and sample 2- with diabetes) after storage of samples in treated Eppendorf tubes according to the invention and the untreated Eppedorf tubes. Higher number of vesicles observed in case of storage in untreated Eppendorf tube are due to higher sticking of microvesicles on the walls of Eppendorf tubes and thus exposure of these microvesicles to higher mechanical stress which leads to its fragmentation.

## Claims

1. Method for treating tools used for collecting, sampling, storing, transporting and isolating of microvesicles, nanovesicles and exosomes from mammal blood, said method comprising the steps of:
- Selecting a tool from a plurality of tools comprising needles, blood tubing, blood bags, catheters, Eppendorf tubes, pipettes, or blood bags;
- Providing said tool from said plurality of tools, wherein said tool is made from a polymeric material;
- Providing a source of highly ionized gas of positively and negatively charged particles of high density;
- Treating a polymeric surface of said tool by applying short pulses of said source of particles next to or on said surface of said tool to assure surface modification of said surface by said positively and negatively charged particles of high density by guiding the source of particles along the surface of the tool to be treated in order to increase roughness of the surface;
- wherein said treating step is performed in a dry state under atmospheric pressure;
- wherein said source provides a density of charged particles next to or on the surface between 10¹⁶ m⁻³ and 10²⁰ m⁻³, or between 1x10¹⁷ m⁻³ and 1x10¹⁹ m⁻³, or between 3x10¹⁷ m⁻³ and 3x10¹⁸ m⁻³; and
- wherein said source provides short pulses of time span between 1 and 1000 ns, or between 10 and 100 ns, or 30 ns.

2. Method according to claim 1, wherein said mammal may be human, equine, bovine, murine, feline, porcine, canine or ovine.

3. Method according to any of the preceding claims, wherein the surfaces of said tools used for collecting, sampling, storing, transporting and isolating of microvesicles, nanovesicles and exosomes are exposed to multiple number of pulses between 1 to 10⁶, or between 100 to 10,000 pulses.

4. Method according to any of the preceding claims, wherein the frequency of said pulses is between 10 and 10⁶ Hz, or between 0.1 and 100 kHz.

5. Method according to any of the preceding claims, wherein said polymeric material is selected from the group consisting of biopolymers, synthetic polymers, polyethylene, polypropylene, polyurethane, polyterpene, inorganic polymers, phenolic resins, polyanhydrides, polypropylene, polystyrene, polyolefins, polyalkenes, polyamide, polyacetal, polyethylene terephthalate, polycarbonate, and cellulose acetate.

6. Tools for collecting, sampling, storing, transporting and isolating of microvesicles, nanovesicles and/or exosomes from mammal blood, wherein said tools are treated according to a method according to any of the preceding claims.

7. Use of tools according to claim 6 for collecting, sampling, storing, transporting and isolating of microvesicles, nanovesicles and/or exosomes from mammal blood.

## Patentansprüche

1. Verfahren zur Behandlung von Werkzeugen, die zur Sammlung, zur Probennahme, zur Lagerung, zum Transport und zur Isolierung von Mikrovesikeln, Nanovesikeln und Exosomen aus Säugetierblut verwendet werden, wobei das Verfahren folgende Schritte umfasst:
- Wählen eines Werkzeugs aus einer Vielzahl von Werkzeugen, umfassend Nadeln, Blutschläuche, Blutbeutel, Katheter, Eppendorf-Röhrchen, Pipetten oder Blutbeutel;
- Bereitstellen des Werkzeugs aus einer Vielzahl von Werkzeugen, wobei das Werkzeug aus einem Polymermaterial besteht;
- Bereitstellen einer Quelle von stark ionisiertem Gas von positiv und negativ geladenen Teilchen hoher Dichte;
- Behandeln einer Polymeroberfläche des Werkzeugs durch Aufbringen von kurzen Impulsen der Partikelquelle neben oder auf der Oberfläche des Werkzeugs zur Gewährleistung von Oberflächenmodifizierung der Oberfläche durch die positiv und negativ geladenen Teilchen hoher Dichte durch Führen der Partikelquelle entlang der Oberfläche des zu behandelnden Werkzeugs zur Erhöhung der Rauheit der Oberfläche;
- wobei der Behandlungsschritt in trockenem Zustand unter Atmosphärendruck durchgeführt wird;
- wobei die Quelle eine Dichte von geladenen Teilchen neben oder auf der Oberfläche zwischen 10¹⁶ m⁻³ und 10²⁰ m⁻³ oder zwischen 1 x 10¹⁷ m⁻³ und 1 x 10¹⁹ m⁻³ oder zwischen 3 x 10¹⁷ m⁻³ und 3 x 10¹⁸ m⁻³ bereitstellt; und
- wobei die Quelle kurze Impulse mit einer Zeitspanne zwischen 1 und 1000 ns oder zwischen 10 und 100 ns oder 30 ns bereitstellt.

2. Verfahren nach Anspruch 1, wobei es sich bei dem Säugetier um einen Menschen, ein Pferd, ein Rind, eine Maus, eine Katze, ein Schwein, einen Hund oder ein Schaf handeln kann.

3. Verfahren nach einem der vorhergehenden Ansprüche, wobei die Oberflächen der Werkzeuge, die zur Sammlung, zur Probennahme, zur Lagerung, zum Transport und zur Isolierung von Mikrovesikeln, Nanovesikeln und Exosomen verwendet werden, mehreren Impulsen zwischen 1 bis 10⁶ oder zwischen 100 bis 10.000 Impulsen ausgesetzt werden.

4. Verfahren nach einem der vorhergehenden Ansprüche, wobei die Frequenz der Impulse zwischen 10 und 10⁶ Hz oder zwischen 0,1 und 100 kHz liegt.

5. Verfahren nach einem der vorhergehenden Ansprüche, wobei das Polymermaterial aus der Gruppe bestehend aus Biopolymeren, synthetischen Polymeren, Polyethylen, Polypropylen, Polyurethan, Polyterpen, anorganischen Polymeren, Phenolharzen, Polyanhydriden, Polypropylen, Polystyrol, Polyolefinen, Polyalkenen, Polyamid, Polyacetal, Polyethylenterephthalat, Polycarbonat und Celluloseacetat ausgewählt ist.

6. Werkzeuge zur Sammlung, zur Probennahme, zur Lagerung, zum Transport und zur Isolierung von Mikrovesikeln, Nanovesikeln und/oder Exosomen aus Säugetierblut, wobei die Werkzeuge gemäß einem Verfahren nach einem der vorhergehenden Ansprüche behandelt worden sind.

7. Verwendung von Werkzeugen nach Anspruch 6 zur Sammlung, zur Probennahme, zur Lagerung, zum Transport und zur Isolierung von Mikrovesikeln, Nanovesikeln und/oder Exosomen aus Säugetierblut.

## Revendications

1. Méthode de traitement d'outils utilisés pour la collecte, l'échantillonnage, le stockage, le transport et l'isolement de microvésicules, de nanovésicules et d'exosomes issus de sang mammalien, ladite méthode comprenant les étapes consistant à :
- sélectionner un outil parmi une pluralité d'outils comprenant les aiguilles, les tubulures pour le sang, les poches de sang, les cathéters, les tubes Eppendorf, les pipettes ou les poches de sang ;
- fournir ledit outil issu de ladite pluralité d'outils, où ledit outil est fait à partir d'un matériau polymère ;
- fournir une source de gaz hautement ionisé de particules de haute densité, chargées positivement et négativement ;
- traiter une surface polymère dudit outil par l'application de courtes impulsions de ladite source de particules à proximité ou sur ladite surface dudit outil, afin de garantir une modification de surface de ladite surface par lesdites particules de haute densité, chargées positivement et négativement, en guidant la source de particules le long de la surface de l'outil à traiter, afin d'augmenter la rugosité de la surface ;
- où ladite étape de traitement est effectuée à l'état sec et sous pression atmosphérique ;
- où ladite source fournit une densité de particules chargées à proximité ou sur la surface comprise entre 10¹⁶ m⁻³ et 10²⁰ m⁻³, ou entre 1 × 10¹⁷ m⁻³ et 1 × 10¹⁹ m⁻³, ou entre 3 × 10¹⁷ m⁻³ et 3 × 10¹⁸ m⁻³ ; et
- où ladite source fournit des impulsions courtes dans un laps de temps compris entre 1 et 1000 ns, ou entre 10 et 100 ns, ou de 30 ns.

2. Méthode selon la revendication 1, dans laquelle ledit mammifère peut être humain, équin, bovin, murin, félin, porcin, canin ou ovin.

3. Méthode selon l'une quelconque des revendications précédentes, dans laquelle les surfaces desdits outils utilisés pour la collecte, l'échantillonnage, le stockage, le transport et l'isolement de microvésicules, de nanovésicules et d'exosomes sont exposées à un nombre multiple d'impulsions allant de 1 à 10⁶, ou allant de 100 à 10 000 impulsions.

4. Méthode selon l'une quelconque des revendications précédentes, dans laquelle la fréquence desdites impulsions est comprise entre 10 et 10⁶ Hz, ou entre 0,1 et 100 kHz.

5. Méthode selon l'une quelconque des revendications précédentes, dans laquelle ledit matériau polymère est choisi dans le groupe constitué par les biopolymères, les polymères synthétiques, le polyéthylène, le polypropylène, le polyuréthane, le polyterpène, les polymères inorganiques, les résines phénoliques, les polyanhydrides, le polypropylène, le polystyrène, les polyoléfines, les polyalcènes, le polyamide, le polyacétal, le téréphtalate de polyéthylène, le polycarbonate, et l'acétate de cellulose.

6. Outils destinés à la collecte, l'échantillonnage, le stockage, le transport et l'isolement de microvésicules, de nanovésicules et/ou d'exosomes issus de sang mammalien, lesdits outils étant traités selon une méthode selon l'une quelconque des revendications précédentes.

7. Utilisation d'outils selon la revendication 6, pour la collecte, l'échantillonnage, le stockage, le transport et l'isolement de microvésicules, de nanovésicules et/ou d'exosomes issus de sang mammalien.
